# EUROPEAN PATENT SPECIFICATION

(11) **EP 4 412 666 B1**
(45) Date of publication and mention of the grant of the patent: **23.04.2025**
(21) Application number: 22793435.3
(22) Date of filing: 27.09.2022
(51) Int. Cl.: A61L 9/18, H01J 65/00, H01J 65/04

(54) **LIGHTING DEVICE USING MICROWAVE RADIATION**
BELEUCHTUNGSVORRICHTUNG UNTER VERWENDUNG VON MIKROWELLENSTRAHLUNG
DISPOSITIF D'ÉCLAIRAGE UTILISANT LE RAYONNEMENT MICRO-ONDES

(30) Priority: 07.10.2021 EP 21201501
(43) Date of publication of application: 14.08.2024
(73) Proprietor: Signify Holding B.V., 5656 AE Eindhoven (NL)
(72) Inventor: SEPKHANOV, Ruslan, Akhmedovich, 5656 AE Eindhoven (NL); DE SAMBER, Marc, Andre, 5656 AE Eindhoven (NL)
(74) Representative: Verweij, Petronella Daniëlle
(86) International application number: PCT/EP2022/076783
(87) International publication number: WO 2023/057257

(56) References cited:
- EP-A1- 1 947 678
- EP-A1- 3 888 699
- EP-A2- 2 381 463
- CN-U- 213 835 990
- US-A- 5 931 557
- US-A1- 2011 085 147

## Description

### FIELD OF THE INVENTION

The invention relates to a lighting device. The invention further relates to an array of lighting devices.

### BACKGROUND OF THE INVENTION

Recent pathogen outbreaks have shown that there is an increased demand for more powerful, more efficient, faster, cheaper, and more readily available methods to fight infectious diseases. Pathogens such as viruses can be transmitted via short distance particle transmission between humans, e.g., during coughing or sneezing, but also significantly via aerosols containing virus particles. The aerosols are generated by humans during normal human functioning such as breathing and talking. Aerosol droplets containing virus particles can be present in air for a rather long time, posing danger for people present in the space. Mitigation methods often include disinfection devices installed within the air circulation systems. These devices filter the air, deactivating the virus particles within the aerosol droplets before returning the air back to the space, thus disinfecting the air.

A recent disinfection method is the introduction of ultraviolet-C, UV-C, based light disinfection systems, mainly based on conventional UV-C light tubes, Excimer lamps, or Xenon lamps. In such systems, the air (and the aerosol droplets that it contains) is treated by the UV-C radiation. However, exposure to UV-C disinfection light above a threshold dose limit is very harmful for humans, potentially resulting in injury to eyes and/or skin. Therefore, even when UV-C light is concealed within an air conditioning/circulation system, it can pose some risk of leaking and damaging people's health. Even in the case such a system is designed with most safety precautions and lowest risk of UV-C light leakage, it can still be perceived as dangerous by prospective customers.

US5931557 A, US 2011/085147 A1, EP1947678 A1, EP2381463 A2 and CN213835990 U present various applications of microwave powered light sources.

### SUMMARY OF THE INVENTION

It is an objective of the invention to provide a lighting device that provides a safer alternative to the UV-C disinfection systems.

To overcome this concern, in a first aspect of the invention, a lighting device is provided. The lighting device comprises:
a microwave generator for generating microwaves into a volume, wherein the microwaves are for disinfecting the volume;
a lighting load for emitting light for illuminating external objects; and
a housing,
wherein the housing encloses the volume and is configured to prevent the microwaves from passing through the housing and to pass through the light.

The lighting device is used to generate microwaves and at least general illumination light. The microwaves are emitted into a volume. The volume will be disinfected by the microwaves. Because the volume is enclosed by a housing that is configured to prevent the microwaves form passing through the housing, the microwaves are contained in the volume. The housing may be interpreted as a Faraday cage. Because the lighting device also has a lighting load for emitting light for illuminating external objects, the housing needs to be adapted to allow the light to pass through. This may be done by providing at least one opening in the housing that allows light to pass through but prevent the microwaves from leaving the volume.

In a further example, the housing comprises a plurality of openings, wherein each opening of the plurality of openings comprises an effective diameter smaller than 1/10 of a wavelength of the microwaves.

Providing openings in the housing, the light can pass through the housing easily. If the openings have an effective diameter smaller than 1/10 of the wavelength of the microwaves, the microwaves cannot escape the housing and the openings provide a surface large enough to allow the light to pass through the housing in an optimal way.

In a further example, the housing comprises a surface, wherein at least part of said surface comprises a coating for reflecting the microwaves,
wherein said surface is facing the microwave generator, wherein the microwaves are reflected back into the volume.

When the housing is provided with a coating that reflects microwaves the efficiency of generating microwaves in the lighting device can be improved. In order to prevent the microwaves from passing through the housing, the housing may absorb the microwaves. The absorption properties of the housing may depend on the material thickness. The material thickness can be various and such that e.g., downwards the material is thicker to prevent microwaves going downwards and sideways thinner to allow microwaves to escape and disinfect the air in the vicinity of the lamp but without interacting with people. When at least a part of the microwaves is reflected back into the volume, the microwaves can be reused for disinfecting the volume instead of being absorbed by the housing.

An example of a housing with a coating is a thin layer of metal that is transparent for visible light but reflects microwaves, e.g. a nm-thin gold layer. Another example is a closed polymer or glass transparent cover that has a fine pattern of metal lines. Another example is a cover material made from conductive polymers, which may be visible light transparent and also electrically conductive.

In a further example, the housing comprises an electrically conductive material, wherein said electrically conductive materials is configured to prevent the microwaves from passing through the housing.

When the housing is provided with electrically conductive material, the microwaves can be blocked in the most efficient way. Electrically conductive materials are widely available and easy to shape into the desired housing. In addition, electrically conductive materials have very good microwave reflecting capabilities by e.g. reflecting the microwaves back into the volume.

In a further example, the electrically conductive material is metal.

In a further example, the microwave generator is adapted to generate a microwave having a frequency between 300 MHZ and 300GHz.

When microwaves are generated in the range of 300 MHz and 300 GHz, they can be used for disinfection because the pathogens will be damaged or destroyed by microwaves in with these wavelengths.

In a further example, the microwave generator is adapted to be controllable independently from the light source.

It may be desired to independently control the microwave generator from the light source. For example, when the lights are turned off, it may be desired to keep the microwave generator on and vice versa.

In a further example, the lighting device comprises:
an air entry and an air exit allowing air to enter the volume and exit the volume respectively; and
an air flow generator for allowing the air to flow from the air entry to the air exit.

When an air flow generator, e.g. a fan, is used to provide an air flow through the volume, an air inlet and an air outlet may be desired. This allows the air flow through the volume to be regulated. The regulation of the air flow may be linked with the amount of disinfection required. In addition, or alternatively, the regulation of the air flow may be linked with the amount of generated microwaves.

In a further example, the air inlet and the air outlet are placed such that air flows over the light source such that the air provides a cooling of the lighting load.

When the air flow is such that air flows over the light sources, the air flow can provide an active cooling for the light sources, effectively cooling down the light sources and increasing their lifetime.

In a further example, the housing comprises an electrically non-conductive transparent cover.

To prevent any undesired elements from entering the volume, a non-conductive transparent cover can be placed inside or outside to the housing. The housing prevents the microwaves from leaving the volume and the cover prevents anything else from entering the volume. Preferably in this case, a dedicated air inlet and air outlet are provided.

In a further example, the microwave generator is adapted to generate a microwave with a frequency of 8.2 GHz.

The pathogens that need to be damaged or destroyed are most susceptible to microwaves with a wavelength of 8.2 GHz. In this example, 8.2 GHz is the most effective frequency for destroying an influenza virus. The microwave generator may provide multiple microwaves at multiple frequencies so that multiple pathogens can be damaged or destroyed.

In a further example, air is provided to the volume through the housing at a first location and wherein the apparatus further comprises an ionizer coupled to the first location for ionizing the air entering the volume to a first potential and wherein the housing is adapted to be set to the first potential.

In addition to the microwave generator, an ionizer can be used. The ionizer generates ions in the air flow. These ions may damage or destroy at least part of the pathogens and/or carrier molecules conveying the pathogens. In addition, particles in the air potentially carrying the pathogens are charged to the same potential as the housing and therefore less collisions between the particles and the housing will occur.

In a further example, the lighting load comprises a light emitting diode, LED.

Preferably, LEDs are used as the lighting load because they convert electric energy into visible light very efficiently.

In a further example, the lighting device further comprises a presence sensor for detecting a presence in a space, wherein upon detection of a presence, the microwave generator is activated.

A presence sensor may be used to turn on the microwave generated upon detection of a person entering a room. A room can be a dedicated place within a building, but any kind of environment or volume can also be understood as being defined as a room. It may be desired to only disinfect air entering the volume when a presence is detected. When nobody is present in the room, the microwave generator may be turned off since it may not be required to disinfect. Alternatively, or additionally, the lighting load may also respond to the presence detection.

In another example, an array of lighting devices is provided. The array of lighting devices is arranged in a grid.

When multiple lighting devices are used and placed in a grid, a larger volume of air can be effectively disinfected.

### BRIEF DESCRIPTION OF THE DRAWINGS

Examples of the invention will now be described with reference to the accompanying drawings, in which:
Fig. 1 shows an example of a lighting device.
Fig. 2 shows an improved example of a lighting device.
Fig. 3 shows a further improved example of a lighting device.
Fig. 4 shows a further improved example of a lighting device.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should also be understood that the Figures are merely schematic and are not drawn to scale. It should also be understood that the same reference numerals are used throughout the Figures to indicate the same or similar parts.

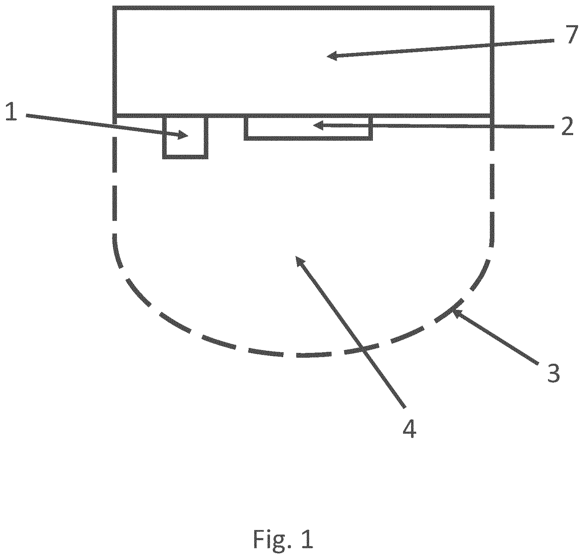

Figure 1 shows an example of a lighting device. The lighting device has a microwave generator 1 that is used for generating microwave radiation. The lighting device also has a lighting load 2, which emits light for illuminating external objects. The lighting device can therefore be used for general illumination e.g. for illuminating at least a part of a room. The lighting device has a housing 3 which encloses a volume 4. The housing 3 can be part of a luminaire, in which all components of the luminaire are comprised. This means that in the example of a luminaire, the housing 3 encloses the microwave generator 1 and the lighting load 2. The microwave generator 1 and the lighting load 2 may be controlled by a control unit 7. This control unit 7 may also be enclosed by the housing 3. The control unit 7 may control the power provided to the microwave generator 1 and the lighting load 2. This control unit 7 may provide for an independent power control to the microwave generator 1 and the lighting load 2 such they can be controlled independently from each other. A major advantage of placing the microwave generator 1 in a lighting device with a lighting load 2 is that the microwave generator 1 will be located close to the place where it is needed to provide disinfection, namely in the vicinity of people. People require lighting in a room and the addition of the microwave generator 1 allows a disinfection of the air in this room. This allows people in the room to be provided with good lighting and a good air quality. The housing 3 is preferably configured to prevent the microwave radiation generated by the microwave generator 1 from leaving the volume 4 so that the people located nearby the lighting device will not be exposed to the microwave radiation. It is to be noted that even if some microwave radiation is leaked into the room, this will not be harmful for any people that are present in the room. In contrast, UV-C that leaks into the room would be harmful for people. It is therefore to be noted that disinfecting with microwave radiation is significantly safer than disinfecting with UV-C. The present invention is advantageous for people, but may mutatis mutandis apply to animals in an agricultural context, and confer the same advantages. The housing 3 may behave like a Faraday cage. In addition, the housing 3 is configured to provide the illumination by the lighting load 2 to the external objects in the room. Preferably, the housing 3 has a plurality of openings that allows the light generated by the lighting load 2 to leave the housing 3. To prevent the microwaves generated by the microwave generator 1 from leaving the housing 3, a maximum size of the opening may not exceed the wavelength of the microwaves. Preferably, the maximum size of the openings in the housing 3 is smaller than or equal to 1/10 of the wavelength of the microwaves. This allows an optimum blocking of the microwaves by the housing 3, while also providing good openings for the light to pass through. The housing 3 with the openings can be configured in a mesh so that an evenly distribution of the light is provided to the surroundings e.g. the external objects. The mesh may function as a Faraday cage. The mesh may then look like a lamp shade and the openings in the mesh might be chosen such that microwave leakage is created on purpose, with e.g., more leakage sidewards and low leakage downwards of a ceiling luminaire. For a wall mounted luminaire, the leakage might be created such that only upwards or wall-shearing external microwaves are created.

The microwave generator 1 provides microwaves that are used to disinfect the volume 4. The volume 4 is enclosed by the housing 3. In the volume 4, particles and pathogens are present that are required to be disinfected or destroyed. The particles may be for example aerosol droplets containing virus particles. The microwave generator 1 generates microwaves in the volume 4 so that the particles are subjected to the microwaves. The particles absorb the energy that is present in the microwaves. By absorbing the energy from the microwaves, the virus or pathogens in the particles becomes deactivated. The microwave energy is transferred to virions by the process referred to as the structure resonant energy transfer (SRET). A virion can be considered as a homogenous ball. This ball has mechanical modes of vibrations. These modes include the zeroth mode (also referred to as "breathing" mode) where virion expands and contracts in the same way in all directions, the dipole mode where the core and the shell of the virion oscillate with respect to each other's initial positions, the quadrupole mode where the virion gets squeezed alternatingly along two perpendicular directions, etc. From these modes the dipole mode is the one that couples to the incident microwave electromagnetic radiation. The coupling is rather strong, achieving in many cases 100% of energy transfer from the microwave to the mechanical oscillations. The frequency of the microwaves preferably lies between the 300 MHz and 300 GHz. Preferably a microwave frequency of 8.2 GHz is used. At this frequency, the virus particles resonate best and absorb most of the power. It is therefore at this frequency that the microwave generator 1 requires a minimum power for deactivating the virus.

For the housing 3 to prevent the microwaves from leaving the volume 4, the housing 3 may be adapted to absorb the microwaves. This may be done with an electrically non-conductive material such as plastic. The thickness of the electrically-nonconductive material also may determine how much microwave radiation can leak through the housing 3. It is to be understood that under the definition of preventing the microwaves form leaving the housing 3, it is meant that most of the microwave radiation is not leaving the housing 3 but a leakage of microwave radiation outside the housing 3 may still occur. Electrically conductive material is very well suited for reflecting the microwaves and preventing the microwaves to pass through the housing 3. Preferably, the electrically conductive material is a metal. Metal can absorb microwaves very well and is also very well suited for forming the housing 3. Examples of preferred metals are iron, copper and aluminum.

The housing 3 may be provided with an electrically non-conductive and visible light transparent cover that covers at least a part of the openings than are present in the housing 3. This may be done to prevent any undesired materials to be (accidentally) placed in the volume 4. In addition, it may be prevented that a person can put a finger in the volume 4. It may therefore be desired to cover at least the openings that can be reached after installing the lighting device in with the electrically non-conductive and transparent cover.

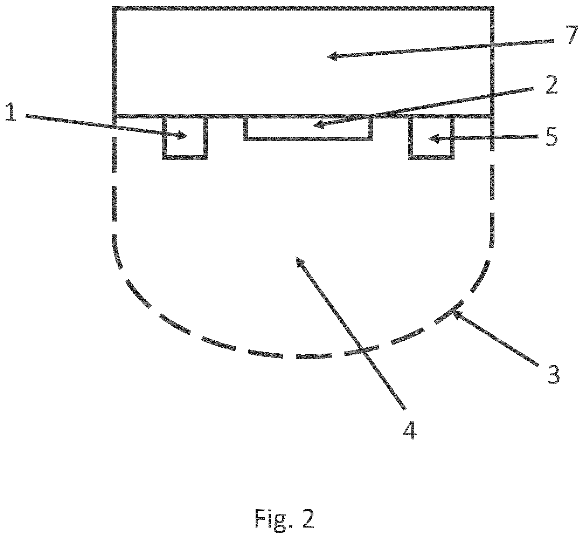

Figure 2 shows an improved example of the lighting device of Figure 1. The lighting device shown in Figure 2 may have all the technical features that have been provided in Figure 1. Preferably, the lighting device has the microwave generator 1, the lighting load 2, the housing 3 and the volume 4. In addition, the lighting device may have a presence sensor 5. The presence sensor 5 may be used to detect presence of a person in a room. The lighting device may be placed in this room. When a presence of the person is detected, the lighting device may respond to this. For example, upon the presence detection, the lighting device may turn on the lighting load 2. In addition, or alternatively, the lighting device may turn on the microwave generator 1. It is preferred to only provide disinfection when people are in the room. Since people are the spreaders of pathogens, it makes sense to only disinfect upon the presence of a person. A person entering the room may also prefer to have the room illuminated. The microwave generator 1 and the lighting load 2 may then be both activated upon the presence detection. A non-limitative list of examples of presence sensors 5 may be: passive infra-red, PIR, sensors, radio detection and ranging, RADAR, sensors, thermopile sensor, or a camera.

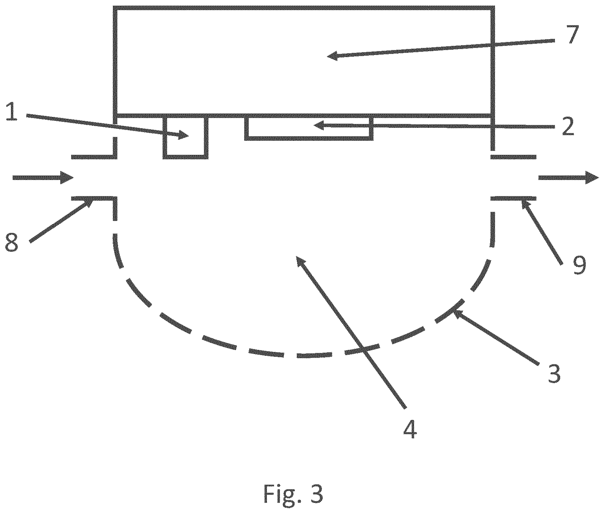

Figure 3 shows another improved example of the lighting device of Figure 1. The lighting device shown in Figure 3 may have all the technical features that have been provided in Figure 1. Preferably, the lighting device has the microwave generator 1, the lighting load 2, the housing 3 and the volume 4. In addition, the lighting device may have a presence sensor 5 as shown in Figure 2. The housing may be provided with an air inlet 8 and an air outlet 9. The use of an air inlet 8 and an air outlet 9 may be desired when a specific air flow is preferred or required. This can for example be the case when the lighting device is part of an air flow system in a building. The air flow system may provide an air flow to the lighting device via the air inlet 8. This may for example be done with a hose coupled to the air inlet 8. The air outlet 9 may then for example provide the disinfected air to the room where people may be present. More generic, the air inlet 8 may be an opening for receiving air that needs to be disinfected. The air outlet 9 may then be used to provide disinfected air to the room. To allow the air to effectively flow from the air inlet 8 to the air outlet 9 and pass through the volume 4, an air flow generator can be used. A non-limitative set of examples of air flow generators may be: fans and ionic wind generation.

Similar to the example provided with Figure 1, the lighting device may also have an electrically non-conductive and transparent cover placed to the housing 3. In this example the electrically non-conductive and transparent cover may be used to allow the air flow to be regulated from the air inlet 8 to the air outlet 9 as the electrically non-conductive and transparent cover may block any air from passing through leaving the air inlet 8 and the air outlet 9 as the most relevant air entry and exit for the volume 4. In the example provided, the air inlet 8 and the air outlet 9 are located such that the airflow is directed towards the lighting load 2. This allows the lighting load 2 to be cooled with the air flow. The air inlet 8 and the air outlet 9 may be placed on opposite ends of the volume 4.

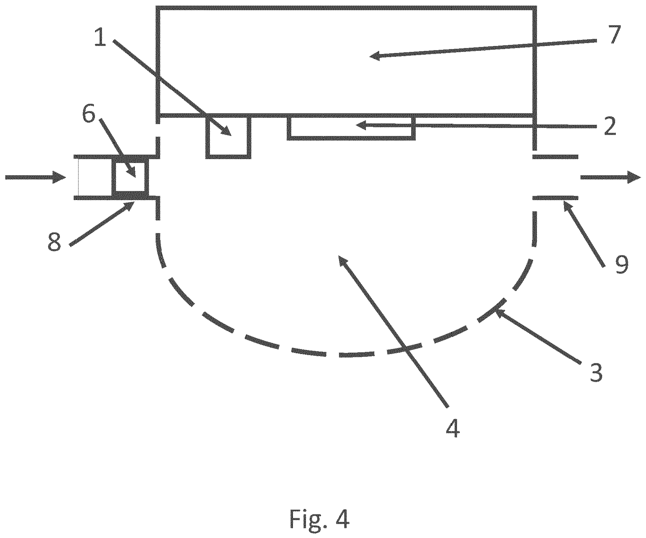

Figure 4 shows another improved example of the lighting device of Figure 1. The lighting device shown in Figure 4 may have all the technical features that have been provided in Figure 1. Preferably, the lighting device has the microwave generator 1, the lighting load 2, the housing 3 and the volume 4. In addition, the lighting device may have a presence sensor 5 as shown in Figure 2. The housing 3 may be provided with an air inlet 8 and an air outlet 9. The air inlet 8 may be provided with an ionizer 6. The ionizer can be used to provide ions in the air flow. The ions will interact with the pathogens and weaken them before they enter the volume 4. The weakened pathogens can then easier be deactivated by the microwave radiation in the volume 4. The ions are generated at a predetermined polarity. Positive or negative ions can be generated. Preferably, the housing 3 is set to an identical polarity as the ions so that the ions will not be attracted to the housing 3 and collisions between the housing 3 and the pathogens are avoided. It may in this example be desired to provide the electrically non-conductive and transparent cover at the outside of the housing 3 so that any contact with the housing 3 from the outside, e.g. by a person, can be avoided.

In the examples provided, the lighting load 2 is used for emitting light. A non-limitative list of examples of lighting loads is: incandescent lamp, fluorescent lamp, high-intensity discharge lamp or light emitting diodes. Preferably, the lighting load has a light emitting diode, LED. LEDs are very energy efficient and can operate well in an environment where microwave radiation is present.

The examples provide in the figures show several embodiments of lighting devices. The lighting devices can be placed in an array so that a larger surface can be illuminated. Simultaneously, a larger volume can be disinfected.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A lighting device comprising:
a microwave generator (1) for generating microwaves into a volume (4), wherein the microwaves are for disinfecting the volume;
a lighting load (2) for emitting light for illuminating external objects; and
a housing (3),
wherein the housing encloses the volume and is configured to prevent the microwaves from passing through the housing and to pass through the light,
**characterized in that** the lighting device further comprises a presence sensor (5) for detecting a presence in a space, wherein upon detection of a presence, the microwave generator is activated.

2. The lighting device according to claim 1, wherein the housing comprises a plurality of openings, wherein each opening of the plurality of openings comprises an effective diameter smaller than 1 /10 or equal to 1/10 of a wavelength of the microwaves.

3. The lighting device according to any of the preceding claims, wherein the housing comprises a surface,
wherein at least part of said surface comprises a coating for reflecting the microwaves,
wherein said surface is facing the microwave generator, wherein the microwaves are reflected back into the volume.

4. The lighting device according to any of the preceding claims, wherein the housing comprises an electrically conductive material, wherein said electrically conductive materials is configured to prevent the microwaves from passing through the housing.

5. The lighting device according to claim 4, wherein the electrically conductive material is metal.

6. The lighting device according to any of the preceding claims, wherein the microwave generator is adapted to generate a microwave having a frequency between 300 MHZ and 300GHz.

7. The lighting device according to any of the preceding claims, wherein the microwave generator is adapted to be controllable independently from the light source.

8. The lighting device according to any of the preceding claims, further comprising:
an air entry (8) and an air exit (9) allowing air to enter the volume and exit the volume respectively; and
an air flow generator for allowing the air to flow from the air entry to the air exit.

9. The lighting device according to claim 8, wherein the air inlet and the air outlet are placed such that air flows over the light source such that the air provides a cooling of the lighting load.

10. The lighting device according to claim 9 wherein the housing comprises an electrically non-conductive transparent cover.

11. The lighting device according to any of the preceding claims, wherein the microwave generator is adapted to generate a microwave with a frequency of 8.2 GHz.

12. The lighting device according to any of the preceding claims, wherein air is provided to the volume through the housing at a first location and wherein the apparatus further comprises an ionizer coupled to the first location for ionizing the air entering the volume to a first potential and wherein the housing is adapted to be set to the first potential.

13. The lighting device according to any of the preceding claims, wherein the lighting load comprises a light emitting diode, LED.

14. An array of lighting devices, each of the lighting devices according to any of the preceding claims, wherein the array of lighting devices is arranged in a grid.

## Patentansprüche

1. Beleuchtungsvorrichtung, umfassend:
einen Mikrowellengenerator (1) zum Erzeugen von Mikrowellen in einem Volumen (4), wobei die Mikrowellen zum Desinfizieren des Volumens dienen;
eine Beleuchtungslast (2) zum Aussenden von Licht zum Beleuchten externer Objekte; und
ein Gehäuse (3),
wobei das Gehäuse das Volumen umschließt und konfiguriert ist, um zu verhindern, dass Mikrowellen durch das Gehäuse hindurchgehen und durch das Licht hindurchgehen,
**dadurch gekennzeichnet, dass** die Beleuchtungsvorrichtung ferner einen Anwesenheitssensor (5) zum Erkennen einer Anwesenheit in einem Raum umfasst, wobei bei Erkennen einer Anwesenheit der Mikrowellengenerator aktiviert wird.

2. Beleuchtungsvorrichtung nach Anspruch 1, wobei das Gehäuse eine Vielzahl von Öffnungen umfasst, wobei jede Öffnung der Vielzahl von Öffnungen einen effektiven Durchmesser aufweist, der kleiner als 1/10 oder gleich 1/10 einer Wellenlänge der Mikrowellen ist.

3. Beleuchtungsvorrichtung nach einem der vorstehenden Ansprüche, wobei das Gehäuse eine Oberfläche umfasst,
wobei mindestens ein Teil der Oberfläche eine Beschichtung zum Reflektieren der Mikrowellen umfasst,
wobei die Oberfläche dem Mikrowellengenerator zugewandt ist, wobei die Mikrowellen in das Volumen zurückreflektiert werden.

4. Beleuchtungsvorrichtung nach einem der vorstehenden Ansprüche, wobei das Gehäuse ein elektrisch leitfähiges Material umfasst, wobei das elektrisch leitfähige Material konfiguriert ist, um zu verhindern, dass Mikrowellen durch das Gehäuse hindurchgehen.

5. Beleuchtungsvorrichtung nach Anspruch 4, wobei das elektrisch leitfähige Material Metall ist.

6. Beleuchtungsvorrichtung nach einem der vorstehenden Ansprüche, wobei der Mikrowellengenerator angepasst ist, um eine Mikrowelle zu erzeugen, die eine Frequenz zwischen 300 MHz und 300 GHz aufweist.

7. Beleuchtungsvorrichtung nach einem der vorstehenden Ansprüche, wobei der Mikrowellengenerator angepasst ist, um unabhängig von der Lichtquelle steuerbar zu sein.

8. Beleuchtungsvorrichtung nach einem der vorstehenden Ansprüche, ferner umfassend:
einen Lufteinlass (8) und einen Luftauslass (9), die es Luft ermöglichen, in das Volumen einzudringen beziehungsweise aus dem Volumen auszudringen; und
einen Luftstromgenerator, zum Ermöglichen der Luft von dem Lufteinlass zu dem Luftauslass zu strömen.

9. Beleuchtungsvorrichtung nach Anspruch 8, wobei der Lufteinlass und der Luftauslass derart platziert sind, dass Luft über die Lichtquelle derart strömt, dass die Luft eine Kühlung der Beleuchtungslast bereitstellt.

10. Beleuchtungsvorrichtung nach Anspruch 9, wobei das Gehäuse eine elektrisch nicht leitfähige transparente Abdeckung umfasst.

11. Beleuchtungsvorrichtung nach einem der vorstehenden Ansprüche, wobei der Mikrowellengenerator angepasst ist, um eine Mikrowelle mit einer Frequenz von 8,2 GHz zu erzeugen.

12. Beleuchtungsvorrichtung nach einem der vorstehenden Ansprüche, wobei dem Volumen an einer ersten Position Luft durch das Gehäuse bereitgestellt wird, und wobei die Einrichtung ferner einen mit der ersten Position gekoppelten lonisator zum Ionisieren der in das Volumen eindringenden Luft auf ein erstes Potential umfasst, und wobei das Gehäuse angepasst ist, um auf das erste Potential eingestellt zu werden.

13. Beleuchtungsvorrichtung nach einem der vorstehenden Ansprüche, wobei die Beleuchtungslast eine Leuchtdiode, LED, umfasst.

14. Anordnung von Beleuchtungsvorrichtungen, wobei jede der Beleuchtungsvorrichtungen einem der vorstehenden Ansprüche entspricht, wobei die Anordnung von Beleuchtungsvorrichtungen in einem Gitter eingerichtet ist.

## Revendications

1. Dispositif d'éclairage comprenant :
un générateur de micro-ondes (1) pour générer des micro-ondes dans un volume (4), dans lequel les micro-ondes sont destinées à désinfecter le volume ;
une charge d'éclairage (2) pour émettre de la lumière afin d'éclairer des objets externes ; et
un boîtier (3),
dans lequel le boîtier renferme le volume et est conçu pour empêcher les micro-ondes de traverser le boîtier et de passer à travers la lumière,
**caractérisé en ce que** le dispositif d'éclairage comprend en outre un capteur de présence (5) pour détecter une présence dans un espace, dans lequel le générateur de micro-ondes est activé lors de la détection d'une présence.

2. Dispositif d'éclairage selon la revendication 1, dans lequel le boîtier comprend une pluralité d'ouvertures, dans lequel chaque ouverture de la pluralité d'ouvertures comprend un diamètre effectif inférieur à 1/10 ou égal à 1/10 d'une longueur d'onde des micro-ondes.

3. Dispositif d'éclairage selon l'une quelconque des revendications précédentes, dans lequel le boîtier comprend une surface,
dans lequel au moins une partie de ladite surface comprend un revêtement destiné à réfléchir les micro-ondes,
dans lequel ladite surface fait face au générateur de micro-ondes, dans lequel les micro-ondes sont réfléchies dans le volume.

4. Dispositif d'éclairage selon l'une quelconque des revendications précédentes, dans lequel le boîtier comprend un matériau conducteur d'électricité, dans lequel ledit matériau conducteur d'électricité est conçu pour empêcher les micro-ondes de traverser le boîtier.

5. Dispositif d'éclairage selon la revendication 4, dans lequel le matériau conducteur d'électricité est un métal.

6. Dispositif d'éclairage selon l'une quelconque des revendications précédentes, dans lequel le générateur de micro-ondes est adapté pour générer une micro-onde présentant une fréquence comprise entre 300 MHZ et 300 GHz.

7. Dispositif d'éclairage selon l'une quelconque des revendications précédentes, dans lequel le générateur de micro-ondes est adapté pour être commandé indépendamment de la source de lumière.

8. Dispositif d'éclairage selon l'une quelconque des revendications précédentes, comprenant en outre :
une entrée d'air (8) et une sortie d'air (9) permettant à l'air d'entrer dans le volume et d'en sortir respectivement ; et
un générateur de flux d'air pour permettre à l'air de circuler de l'entrée d'air à la sortie d'air.

9. Dispositif d'éclairage selon la revendication 8, dans lequel l'entrée d'air et la sortie d'air sont placées de telle sorte que l'air circule au-dessus de la source de lumière, de telle sorte que l'air assure un refroidissement de la charge d'éclairage.

10. Dispositif d'éclairage selon la revendication 9, dans lequel le boîtier comprend un couvercle transparent non conducteur d'électricité.

11. Dispositif d'éclairage selon l'une quelconque des revendications précédentes, dans lequel le générateur de micro-ondes est adapté pour générer une micro-onde à une fréquence de 8,2 GHz.

12. Dispositif d'éclairage selon l'une quelconque des revendications précédentes, dans lequel l'air est fourni au volume à travers le boîtier au niveau d'un premier emplacement et dans lequel l'appareil comprend en outre un ionisateur couplé au premier emplacement pour ioniser l'air entrant dans le volume à un premier potentiel et dans lequel le boîtier est adapté pour être réglé au premier potentiel.

13. Dispositif d'éclairage selon l'une quelconque des revendications précédentes, dans lequel la charge d'éclairage comprend une diode électroluminescente, DEL.

14. Réseau de dispositifs d'éclairage, chacun des dispositifs d'éclairage selon l'une quelconque des revendications précédentes, dans lequel le réseau de dispositifs d'éclairage est agencé dans une grille.
